# EUROPÄISCHE PATENTSCHRIFT

(11) **EP 3 920 860 B1**
(45) Veröffentlichungstag und Bekanntmachung des Hinweises auf die Patenterteilung: **29.05.2024**
(21) Anmeldenummer: 20702297.1
(22) Anmeldetag: 31.01.2020
(51) Int. Cl.: A61F 13/00, C08G 18/48, C08G 18/76, C08G 18/10, C08L 75/08, C08G 101/00

(54) **POLYURETHANWEICHSCHAUMSTOFFE MIT HOHER WASSERAUFNAHMEFÄHIGKEIT**
FLEXIBLE POLYURETHANE FOAMS HAVING HIGH WATER ABSORBENCY
MOUSSES SOUPLES DE POLYURÉTHANE À CAPACITÉ D'ABSORPTION DE L'EAU ÉLEVÉE

(30) Priorität: 07.02.2019 EP 19155983
(43) Veröffentlichungstag der Anmeldung: 15.12.2021
(73) Patentinhaber: BASF SE, 67056 Ludwigshafen am Rhein (DE)
(72) Erfinder: SCHUETTE, Markus, 49448 Lemfoerde (DE); LUTTER, Heinz-Dieter, 49448 Lemfoerde (DE); SCHUBERT, Anne, 49448 Lemfoerde (DE)
(74) Vertreter: BASF IP Association
(86) Internationale Anmeldenummer: PCT/EP2020/052407
(87) Internationale Veröffentlichungsnummer: WO 2020/161010

(56) Entgegenhaltungen:
- EP-A1- 2 336 211
- US-A- 5 591 779
- US-A1- 2005 176 840

## Beschreibung

Die vorliegende Erfindung betrifft ein Verfahren zur Herstellung eines hydrophilen Polyurethanweichschaumstoffs, bei dem man (a) mindestens ein Polyurethanprepolymer mit einem Isocyanatgehalt von 6 bis 10 Gew.-%, bezogen auf das Gewicht des Isocyanatprepolymers (a), mit (b) mindestens einer wässrigen Kompoente im Gewichtsverhältnis Polyurethanprepolymer (a) zu wässriger Komponente (b) von 3 zu 1 bis 1 zu 1,2 vermischt und zum Polyurethanweichschaumstoff ausreagieren lässt, wobei das Polyurethanprepolymer (a) erhältlich ist durch Vermischen und Umsetzen mindestens eines Isocyanats (a1) mit mindestens einem Polyetherol (a2), das Isocyanat (a1) Methylendiphenylendiisocyanat enthält und das Polyetherol (a2) eine Hydroxylzahl von 30 bis 60 mg KOH/g aufweist, erhältlich ist durch Alkoxylierung mindestens eines difunktionellen und/oder trifunktionellen Startermoleküls mit Ethylenoxid und Propylenoxid und der Gehalt an Ethylenoxid, bezogen auf das Gesamtgewicht an Alkylenoxid, mindestens 60 Gew.-%, bezogen auf das Gesamtgewicht des Polyetherpolyols (a2), beträgt, wobei das Polyetherol (a2) erhältlich ist durch Propoxylierung des oder der Startermoleküle in einem ersten Schritt bis zu einer Hydroxylzahl von 400 bis 1200 mg KOH/g, anschließend einer Alkoxylierung des propoxylierten Startermoleküls mit einer Mischung von Ethylenoxid und Propylenoxid und schließlich einer Ethoxylierung des so erhaltene Alkoxylierungsprodukts mit 2 bis 10 Gew.-% Ethylenoxid, bezogen auf das zur Herstellung des Polyetherols (a2) eingesetzte Alkylenoxid, sowie die wässrige Komponente (b) mindestens 90 Gew.-% Wasser (b1) und bis zu 10 Gew.-% nicht silikonhaltige oberflächenaktive Substanzen (b2) enthält. Weiter betrifft die vorliegende Erfindung einen Polyurethanweichschaumstoff, erhältlich nach einem solchen Verfahren sowie die Verwendung eines solchen Polyurethanweichschaumstoffs zur Wundbehandlung.

Der Einsatz von hydrophilen Polyurethanweichschaumstoffen zur Behandlung von Wunden ist bekannt und beispielsweise in WO 2012055834, WO 2004074343, EP 2336211, US 5591779 und WO 9429361 beschrieben.

WO 2012055834 und EP 2336211 offenbaen die Herstellung von hydrophilen Polyurethanweichschaumstoffen ausgehend von aliphatischen Isocyanaten. Dazu wird ein Isocyanatprepolymer mit einer wässrigen Komponente umgesetzt. Nachteile solcher aliphatischer Isocyanate ist deren hohe Flüchtigkeit und damit die davon ausgehende Gesundheitsgefahr beim Verarbeiten. Weiter sind aliphatische Isocyanate bei der Verarbeitung nur wenig reaktiv, woraus energieintensive und langsame Prozesse resultieren. Dabei ist eine Beschleunigung durch den Einsatz von Katalysatoren problematisch, da diese aus dem Schaumstoff migrieren können und ein solcher Schaumstoff nicht zur Wundbehandlung geeignet wäre.

WO 2004074343 und WO 9429361 beschreiben hydrophile Polyurethanweichschaumstoffe, die durch Umsetzen von Polyurethanprepolymeren auf Basis von MDI und hydrophilen Polyetherolen, mit einer wässrigen Komponente. Die in diesen Dokumenten beschriebenen Verfahren führen allerdings zu Polyurethanweichschaumstoffen mit verbesserungswürdiger Wasseraufnahme, und zwar bezogen auf die absolute Menge an aufgenommenem Wasser und der Wasseraufnahmegeschwindigkeit sowie einer verbesserungsfähigen Nassreißfestigkeit.

US 2005176840 betrifft ein Verfahren zur Herstellung von PU-Weichschäumen beinhaltend ein NCO-terminiertes Prepolymer auf MDI/Polyalkylenoxid-Basis, wobei das Polyalkylenoxid die Struktur -PO-PO/EO-EO aufweist. Dabei wird ein MDI basiertes Prepolymer eingesetzt, das neben MDI ein Polyethylen-Polypropylen basiertes Polyetherpolyol mit einem Molekulargewicht von 2000 bis 10.000g/mol und einer Funktionalität von 2 bis 6 aufweist, wobei der Oxyethylenanteil 21 bis 45 Gew.-% und der Oxypropylenanteil 60 b is 90 Gew.-% beträgt. Dieses wird unter Verwendung weiterer Polyole und eines silikonbasierten Tensids zu einem PU-Weichschaum umgesetzt.

US 5591779 offenbart die Herstellung eines NCO-Prepolymers beinhaltend MDI und Polyethertriol auf EO/PO-Basis, welches mit einer wässrigen Komponente, beinhaltend ein EO/PO-Tensid zu einem POlyurethan-Weichschaum mit einer Dichte von 64-170 kg/m³ ungesetzt wird.

Aufgabe der vorliegenden Erfindung war es daher Polyurethanweichschaumstoffe zu liefern, die zur Behandlung von Wunden geeignet sind, wobei die mechanischen Eigenschaften, insbesondere die Nassreißfestigkeit, sowie die Wasseraufnahme gegenüber bekannten hydrophilen Polyurethanweichschaumstoffen weiter verbessert ist.

Die erfindungsgemäße Aufgabe wird gelöst durch einen Polyurethanweichschaumstoff, erhältlich durch ein Verfahren, bei dem man (a) mindestens ein Polyurethanprepolymer mit einem Isocyanatgehalt von 6 bis 10 Gew.-%, bezogen auf das Gewicht des Isocyanatprepolymers (a), mit (b) mindestens einer wässrigen Komponente im Gewichtsverhältnis Polyurethanprepolymer (a) zu wässriger Komponente (b) von 3 zu 1 bis 1 zu 1,2 vermischt und zum Polyurethanweichschaumstoff ausreagieren lässt, wobei das Polyurethanprepolymer (a) erhältlich ist durch Vermischen und Umsetzen mindestens eines Isocyanats (a1) mit mindestens einem Polyetherol (a2), das Isocyanat (a1) Methylendiphenylendiisocyanat enthält und das Polyetherol (a2) eine Hydroxylzahl von 30 bis 60 mg KOH/g aufweist, erhältlich ist durch Alkoxylierung mindestens eines difunktionellen und/oder trifunktionellen Startermoleküls mit Ethylenoxid und Propylenoxid und der Gehalt an Ethylenoxid, bezogen auf das Gesamtgewicht an Alkylenoxid, mindestens 60 Gew.-%, bezogen auf das Gesamtgewicht des Polyetherpolyols (a2), beträgt, wobei das Polyetherol (a2) erhältlich ist durch Propoxylierung des oder der Startermoleküle in einem ersten Schritt bis zu einer Hydroxylzahl von 400 bis 1200 mg KOH/g, anschließend einer Alkoxylierung des propoxylierten Startermoleküls mit einer Mischung von Ethylenoxid und Propylenoxid und schließlich einer Ethoxylierung des so erhaltene Alkoxylierungsprodukts mit 2 bis 10 Gew.-% Ethylenoxid, bezogen auf das zur Herstellung des Polyetherols (a2) eingesetzte Alkylenoxid, sowie die wässrige Komponente (b) mindestens 90 Gew.-% Wasser (b1) und bis zu 10 Gew.-% nicht silikonhaltige oberflächenaktive Substanzen (b2) enthält. Weiter betrifft die vorliegende Erfindung einen Verfahren zur Herstellung eines solchen Polyurethanweichschaumstoffs und dessen Verwendung zur Wundbehandlung.

Der erfindungsgemäße Weichschaumstoff weist vorzugsweise eine Dichte von 70 bis 140 g/l, besonders bevorzugt 80 bis 120 g/L und insbesondere 85 bis 115 g/L auf. Dabei wurde im Rahmen der vorliegenden Erfindung die Dichte nach Annex C des europäischen Standards EN 14315-2 bestimmt. Weiter weisen erfindungsgemäße Polyurethanweichschaumstoffe vorzugsweis eine Härte von 1,0 bis 5,0, besonders bevorzugt von 2,5 bis 3,5 auf. Dabei wurde im Rahmen der vorliegenden Erfindung die Härte nach Asker C ASTM D 2240 bestimmt.

Die erfindungsgemäßen Polyurethanweichschaumstoffe zeichnen sich weiter durch eine hervorragende Wasseraufnahmefähigkeit aus. Diese wurde wie in den Beispielen erläutert ermittelt und beträgt vorzugsweise mehr als 10 g pro Gramm Schaumstoff, besonders bevorzugt 12 bis 20 g pro Gramm Schaumstoff.

Zur Herstellung des erfindungsgemäßen Polyurethanweichschaumstoffs werden mindestens ein Polyurethanprepolymer (a) mit einem Isocyanatgehalt von 6 bis 10 Gew.-%, vorzugsweise 6 bis 9 Gew.-% und insbesondere 6,5 bis 8,5 Gew.-% bezogen auf das Gewicht des Isocyanatprepolymers (a), mit mindestens einer wässrigen Komponente (b) vermischt und zum Polyurethanschaumstoff umsetzt. Die Bestimmung des Isocyanatgehalts ist bekannt und kann beispielsweise durch Titration oder spektrometrisch erfolgen. Dieser wird im Rahmen der Erfindung auf 0,1 Gew.-% genau angegeben.

Das Isocyanatprepolymer wird dabei erhalten durch Vermischen und Umsetzen mindestens eines Isocyanats (a1) mit mindestens einem Polyetherol (a2).

Als Isocyanate (a) werden vorzugsweise aromatische Di- und Polyisocyanate eingesetzt, diese sind in der Polyurethanchemie bekannt und umfassen beispielsweise Isomere des Toluoldiisocyanats und isomere sowie höherkernige Homologe des Methylendiphenylendiisocyanat. Dabei ist es erfindungswesentlich, dass die Isocyanate Methylendiphenylendiisocyanat (a1) enthalten. Dabei umfasst Methylendiphenylendiisocyanat (im Folgenden auch als MDI bezeichnet, die Isomere 2,2'-MDI, 2,4'-MDI und 4,4'-MDI. Vorzugsweise beträgt der Anteil an 4,4` Methylendiphenylendiisocyanat 30 bis 100 Gew.-% und mehr bevorzugt 45 bis 90 Gew.-%, besonders bevorzugt 55 bis 85 Gew.-%, und der Anteil an 2,4` Methylendiphenylendiisocyanat 0 bis 70 Gew.-% und mehr bevorzugt 10 bis 55 Gew.-% und besonders bevorzugt 15 bis 45 Gew.-%, jeweils bezogen auf das Gesamtgewicht des Isocyanats (a). In einer besonders bevorzugten Ausführungsform enthält das Isocyanat (a), bezogen auf das Gesamtgewicht der Isocyanate (a), weniger als 10 Gew.-% bevorzugt weniger als 5 Gew.-% und insbesondere keine Isocyanate, die von 4,4` Methylendiphenylendiisocyanat und 2,4`-Methylendiphenylendiisocyanat verschieden sind. Insbesondere enthält das Isocyanat (a) weniger als 1 Gew.-% bevorzugt weniger als 0,01 Gew.-% und weiter bevorzugt kein 2,2' Methylendiphenylendiisocyanat.

Als Polyetherole (a2) werden ein oder mehrere Polyetherole mit einer Hydroxylzahl von 30 bis 60 mg KOH/g, vorzugsweise 35 bis 50, eingesetzt, welche erhältlich sind durch Alkoxylierung mindestens eines difunktionellen und/oder trifunktionellen Startermoleküls mit Ethylenoxid und Propylenoxid, der Gehalt an Ethylenoxid, bezogen auf das Gesamtgewicht an Alkylenoxid, mindestens 60 Gew.-%, bevorzugt 65 bis 95 Gew.-% und besonderes bevorzugt 70 bis 80 Gew.-% beträgt, der Gehalt an Propylenoxid größer 0 bis 40 Gew.-%, bevorzugt 5 bis 35 Gew.-% und besonders bevorzugt 20 bis 30 Gew.-% beträgt. Vorzugsweise werden bei der Herstellung des Polyetherols (a2) neben Ethylenoxid und Propylenoxid weniger als 5 Gew.-% und insbesondere keine weiteren Alkylenoxide eingesetzt.

Weiter beträgt erfindungsgemäß der Gehalt an primären Hydroxylgruppendes Polyetherols (a2) mindestens 90 Gew.-%, bevorzugt 95 Gew.-% bis 100 Gew.-%, besonders bevorzugt 99 Gew.-% bis 100 Gew.-% und insbesondere 100%, jeweils bezogen auf das Gesamtgewicht der Hydroxylgruppen des Polyetherpolyols (a2).

Die Polyetherole (a2) können nach bekannten Verfahren hergestellt werden, beispielsweise durch anionische Polymerisation von Alkylenoxiden unter Zusatz mindestens eines Startermoleküls, das vorzugsweise 2 bis 4, besonders bevorzugt 2 oder 3 reaktive Wasserstoffatome gebunden enthält, in Gegenwart von Katalysatoren. Werden Mischungen aus Startermolekülen mit unterschiedlicher Funktionalität eingesetzt, können gebrochenzahlige Funktionalitäten erhalten werden. Einflüsse auf die Funktionalität, beispielsweise durch Nebenreaktionen, werden bei der nominalen Funktionalität nicht berücksichtigt. Als Katalysatoren können Alkalihydroxide, wie Natrium- oder Kaliumhydroxid oder Alkalialkoholate, wie Natriummethylat, Natrium- oder Kaliumethylat oder Kaliumisopropylat, oder bei kationischer Polymerisation Lewis-Säuren, wie Antimonpentachlorid, Bortrifluorid-Etherat oder Bleicherde als Katalysatoren eingesetzt werden. Auch aminische Alkoxylierungs-Katalysatoren, wie Dimethylethanolamin (DMEOA), Imidazol und Imidazolderivate können eingesetzt werden. Weiterhin können als Katalysatoren auch Doppelmetallcyanidverbindungen, sogenannte DMC-Katalysatoren, eingesetzt werden.

Als Startermoleküle kommen Hydroxylgruppen- oder Amingruppenhaltige Verbindungen in Frage, beispielsweise Ethylenglycol, Diethylenglycol, Glycerin, Trimethylolpropan, Pentaerythrit, Methylamin, Ethylamin, Isopropylamin, Butylamin, Benzylamin, Anilin, Toluidin, Toluoldiamin (TDA), Naphtylamin, Ethylendiamin, Diethylentriamin, 4,4'-Methylendianilin, 1,3,-Propandiamin, 1,6-Hexandiamin, Ethanolamin, Diethanolamin, sowie andere zwei oder mehrwertige Alkohole oder ein oder zweiwertige Amine in Betracht. Vorzugsweise ist das Startermolekül zur Herstellung des Polyetherols (a2) ausgewählt ist aus der Gruppe, bestehend aus Ethylenglycol, Diethylenglycol, Propylenglykol, Dipropylenglykol, Glycerin, Trimethylolpropan und Mischungen daraus, besonders bevorzugt wird als Startermolekül Glycerin, insbesondere ausschließlich Glycerin eingesetzt.

Das erfindungsgemäße Polyetherol (a2) ist erhältlich durch Propoxylierung eines Startermolekül in einem ersten Schritt bis zu einer Hydroxylzahl von 400 bis 1200 mg KOH/g, bevorzugt 450 bis 800 mg KOH/g, anschließend mit einer Alkoxylierung des propoxylierten Startermoleküls mit einer Mischung von Ethylenoxid und Propylenoxid und einer Ethoxylierung des so erhaltene Alkoxylierungsprodukts mit 2 bis 10 Gew.-%, bevorzugt 3 bis 5 Gew.-%, Ethylenoxid, bezogen auf das zur Herstellung des Polyetherols (a2) eingesetzte Alkylenoxid. Dabei ist es erfindungswesentlich, dass nach Propoxylierung des Startermoleküls dieses im Mittel mindestens das ein Molekül Propylenoxid aufweist. Werden bereits Propylenoxid haltige Startermoleküle eingesetzt, wie beispielsweise Tripropylenglycol, gilt dieses als bereits propoxyliert, kann aber bis zu einer Hydroxylzahl von 400 mg KOH/g weiter propoxyliert werden. Dadurch wird ein Polyetherol (a2) erhalten, das eine Ethylenoxid-Endgruppe enthält, die 2 bis 10 Gew.%, bevorzugt 3 bis 5 Gew.-% Ethylenoxid, bezogen auf das Gesamtgewicht des eingesetzten Alkylenoxids, enthält. Dabei ist es nicht erforderlich, das propoxylierte Startermolekül nach erfolgter Propoxylierung bzw. das Alkoxylierungsprodukt aus propoxyliertem Startermolekül und der Mischung aus Ethylenoxid und Propylenoxid zu nach erfolgter Alkoxylierung und vor der Ethoyxlierung zu reinigen. Zum Verfolgen des Reaktionsfortschritts kann der Umsatz des Alkylenoxids spektroskopisch verfolgt werden, beispielsweise mittels IR-Spektrometrie. Vor der Zugabe des abschließenden Ethylenoxids wird vorzugsweise mittel Spektroskopischer Methoden der Umsatz von Alkylenoxidüberprüft, damit im Wesentlichen kein nicht umgesetztes Propylenoxid mehr in der reaktionsmischung vorhanden ist. Das bedeutet, dass der Anteil an nicht umgesetztem Propylenoxid vor der Zugabe des Ethylenoxids kleiner als 1 Gew.-%, bevorzugt kleiner als 0,5 Gew.-%, mehr bevorzugt kleiner als 0,1 Gew.-% und insbesondere kleiner als 0,01 Gew.-%, bezogen auf das Gesamtgewicht des bis zu diesem Zeitpunkt eingesetzten Alkylenoxids ist.

Vorzugsweise erfolgt die Aufreinigung der erfindungsgemäßen Polyole (a2) von noch enthaltenem Alkoxylierungskatalysator nach der Herstellung durch in Kontakt bringen mit einem sauren Ionenaustauscher-Harz, beispielsweise Amberlite.

Dabei beträgt der Alkaligehalt des Polyols (a2) vorzugsweise kleiner 50 ppm, besonders bevorzugt kleiner 30 ppm und insbesondere kleiner 20 ppm. Dabei erfolgt die Messung des Alkaligehalts üblicherweise mittels Titration.

Vorzugsweise werden zur Herstellung des Polyisocyanatprepolymers (a) neben dem Polyetherol (a2) keine weiteren Verbindungen mit gegenüber Isocyanat reaktiven Gruppen eingesetzt.

Als wässrige Komponente (b) wird erfindungsgemäß eine Komponente, enthaltend mindestens 90 Gew.-%, vorzugsweise 94 bis 100 Gew.-%, besonders bevorzugt 96 bis 99,5 Gew.-% und insbesondere 97 bis 99 Gew.-% Wasser (b1) und bis zu 10 Gew.-%, bevorzugt 0 bis 6 Gew.-%. Besonders bevorzugt 0,5 bis 4 Gew.-% und insbesondere 1 bis 3 Gew.-% nicht silikonhaltige, oberflächenaktive Substanzen (b2), eingesetzt.

Bei den silikonfreien oberflächenaktiven Substanzen (b2) handelt es sich vorzugsweise um Verbindungen, die bei 20 °C mindestens zu 5 Gew.-% in Wasser löslich sind und die gegenüber Isocyanat reaktive Gruppen aufweisen. Vorzugsweise verringern die oberflächenaktiven Substanzen (b2) bei einer Konzentration von 1 Gew.-% in Wasser bei 20 °C die Oberflächenspannung um mindestens 10 mNm, besonders bevorzugt um 15 mNm und insbesondere um 20 mNm, gemessen nach DIN EN 14370:2004-11 Vorzugsweise werden als oberflächenaktive Substanzen Alkylenoxid-Block-Copolymere mit einen Molekulargewicht von 800 bis 15.000 g/mol, vorzugsweise 1.200 bis 10.000 und insbesondere 2.000 bis 8.000 g/mol. Besonders bevorzugt weisen die Block-Copolymere einen zentralen Block aus Alkylenoxiden mit mindestens 3 Kohlenstoffatomen, wie Propylenoxid, Butylenoxid, Pentylenoxid und Mischungen solcher Alkylenoxide, vorzugsweise Propylenoxid, und jeweils zwei Endblocks aus Ethylenoxid auf. Dabei beträgt das Gewichtsverhältnis von zentralem Block zu Endblocks vorzugsweise 1 zu 0,5 bis 1 zu 5. Solche oberflächenaktiven Substanzen sind unter dem Handelsnamen Pluriol^{®} von der BASF erhältlich.

Neben Wasser (b1) und (oberflächenaktiven Substanzen (b2) enthält die wässrige Komponente (b) vorzugsweise keine Verbindungen, die eine gegenüber Isocyanat reaktiven Gruppen aufweisen, besonders bevorzugt enthält die Komponente (b) keine Aminkatalysatoren und insbesondere gar keine Katalysatoren. In einer weiter bevorzugten Ausführungsform besteht die Komponente (b) nur aus Wasser (b1) und oberflächenaktiven Substanzen (b2).

Zur Herstellung der erfindungsgemäßen hydrophilen Polyurethanweichschaumstoffe werden Polyurethanprepolymer (a) und wässrige Komponente (b) vermischt. Das Vermischen erfolgt vorzugsweise im Gewichtsverhältnis Polyurethanprepolymer (a) zu wässriger Komponente (b) von 2,5 zu 1 bis 1,2 zu 1, besonders bevorzugt 2.2 zu 1 bis 1,0 zu 1 und insbesondere 0,5 zu 1 bis 0,8 zu 1. Das Vermischen erfolgt vorzugsweise bei Temperaturen der Wasserkomponente von größer 0 bis 25 °C, bevorzugt 1 bis 10 °C und insbesondere 3-5°C und Temperaturen der Isocyanat-Komponente bei vorzugsweise 10 bis 50 °C, besonders bevorzugt 15 bis 40 °C und insbesondere 20-35°C. Besonders bevorzugt erfolgt die Aushärtung in Abwesenheit von tertiären Aminen und insbesondere enthält die Mischung aus Polyisocyanatprepolymer und wässriger Komponente (b) keine weiteren Substanzen.

Die Reaktionsmischung wird vorzugsweise kontinuierlich auf ein Trennpapier aufgetragen und ausgehärtet, vorzugsweise im Ofen. Das Auftragen erfolgt üblicherweise in Schichtdicken von 1 mm bis 10 mm. Nach dem Aushärten wird der erfindungsgemäße hydrophile Polyurethanweichschaumstoff von Trennpapier gelöst.

Der erhaltene erfindungsgemäß Polyurethanweichschaumstoff weist eine Dichte von vorzugsweise 70 bis 140 g/l, besonders bevorzugt 80 bis 120 g/L und insbesondere 85 bis 115 g/L und eine Wasseraufnahmefähigkeit von mindestens 8g/g, vorzugsweise mindestens 10 g/g und insbesondere 12 bis 20 g/g auf. Dabei wird die Wasseraufnahmefähigkeit wie Folgt bestimmt: Eine Lösung aus 142 mmol Natriumchlorid und 2,5 mmol Calciumchlorid in 1 Liter entsalztem Wasser wurde verwendet (Nach Methode EN 13726-12002 (Lösung 1)

Der zu prüfende Schaum wird mit einem Stanzeisen eine 50 mm x 50 mm große Schaumstoffprobe aus einem 1,6 cm dicken Schaumstoffstreifen ausgestanzt und 3 Minuten in eine wässrige Testflüssigkeit nach EN 13726-1.2002 1 getaucht. Anschließend wird der Schaumstoff vorsichtig an einer Ecke gefasst ohne diesen auszuwringen, 10 Sekunden abtropfen lassen und erneut gewogen. Die erhaltene Wasseraufnahme (Gewicht Schaumstoff nach dem Abtropfen minus Gewicht der trockenen Schaumstoffprobe) wird durch das Gewicht der trockenen Probe geteilt und in g/g angegeben. Weiter weist der erfindungsgemäße Polyurethanschaumstoff auch eine hohe Wasseraufnahmegeschwindigkeit auf. Zur Bestimmung der Wasseraufnahmegeschwindigkeit wird auf eine trockene Schaumprobe, wie sie auch zu Bestimmung der Wasseraufnahme eingesetzt wird, bei Raumtemperatur 2 ml der oben angegebenen wässrigen Testflüssigkeit auf die Oberfläche der Schaumstoffprobe gegeben und die Zeit gestoppt, bis diese vollständig von der Schaumstoffprobe absorbiert wurde.

Auch weist der erfindungsgemäße Polyurethanschaumstoff sehr gute mechanische Eigenschaften, beispielsweise eine hohe Zugfestigkeit im nassen sowie im trockenen Zustand auf.

Der erfindungsgemäße, hydrophile Polyurethanweichschaumstoff kann vorzugsweise im kosmetischen Bereich, beispielsweise als Kosmetikpads oder Wundauflagen, oder als Schulterpads in Kleidungsstücken eingesetzt werden. Weiter kann der erfindungsgemäße Schaumstoff zur passiven Klimaregulierung in geschlossenen Räumen, beispielsweise in Fahrzeugen, wie Automobilen, oder Gebäuden eingesetzt werden. Auch der Einsatz als reversible Flüssigkeitsaufnahme, beispielsweise in Servierwägen, möglich. Weiter kann der erfindungsgemäße Schaumstoff als Gehörschutz oder zur Aufnahme von Körperflüssigkeiten verwendet werden. Besonders bevorzugt wird der erfindungsgemäße Polyurethanschaumstoff zur Wundbehandlung, beispielsweise als Wundauflage, eingesetzt.

Im Folgenden soll die vorliegende Erfindung anhand von Beispielen verdeutlicht werden.

Die folgenden Substanzen wurden eingesetzt:
Polyol 1: Glycerin gestartetes Polyetherpolyol mit einer Hydroxylzahl von 42 mg KOH/g, erhalten durch in einem ersten Schritt Anlagerung von 6 Gew.-% Propylenoxid, in einem 2. Schritt einer Mischung aus 69 Gew.-% Ethylenoxid und 20 Gew.-% Propylenoxid und in einem dritten Schritt 5 Gew.-% Ethylenoxid mit KOH als Katalysator.
Polyol 2: Diethylenglykol gestartetes Polyetherpolyol mit einer Hydroxylzahl von 42 mg KOH/g, erhalten durch in einem ersten Schritt Anlagerung von 6 Gew.-% Propylenoxid, in einem 2. Schritt einer Mischung aus 69 Gew.-% Ethylenoxid und 20 Gew.-% Propylenoxid und in einem dritten Schritt 5 Gew.-% Ethylenoxid mit KOH als Katalysator.
Polyol 3: Glycerin gestartetes Polyetherpolyol mit einer Hydroxylzahl von 32 mg KOH/g, erhalten durch Alkoxylierung von Glycerin mit einer Mischung aus Ethylenoxid und Propylenoxid, erhältlich unter dem Handelsnamen Voranol ^{®} CP-1421 von der Firma Dow Chemicals.
Polyol 4: Glycerin gestartetes Block Polyetherpolyol mit einer Hydroxylzahl von 42 mg KOH/g, erhalten durch in einem ersten Schritt Anlagerung von 26 Gew.-% Propylenoxid, in einem 2. Schritt 74 Gew.-% Etylenoxid mit KOH als Katalysator.
Hilfsmittel 1: Diglycol-bis-chlorformiat
Hilfsmittel 2: Blockcopolymer aus einem zentralen Polyoxypropylenblock mit einem Molekulargewicht von 1750 g/mol und zwei endständigen Polyoxyethylenblöcken mit einem Molekulargewicht von jeweils 350 g/mol
Hilfsmittel 3: Blockcopolymer aus einem zentralen Polyoxypropylenblock mit einem Molekulargewicht von 1750 g/mol und zwei endständigen Polyoxyethylenblöcken mit einem Molekulargewicht von jeweils 1400 g/mol
wässrige Komponente.: Mischung aus 98 Gew.-% Wasser, 1 Gew.-% Hilfsmittel 2 und 1 Gew.-% Hilfsmittel 3
Iso 1: Mischung enthaltend 98,6 Gew.-% 4,4'-MDI und 1,4 Gew.-% 2,4'-MDI
Iso 2: Mischung aus 2,4 Gew.-% 2,2'-MDI, 48,6 Gew.-% 2,4'-MDI und 49,0 Gew.-% 4,4'-MDI

Prepolymere wurden gemäß Tabelle 1 hergestellt. Dazu wurde das Isocyanat vorgelegt, das Polymer unter Rühren zugetropft und die Mischung bei 80 °C eine Stunde gerührt.

Wenn nicht anders angegeben sind die Mengenangaben in Gewichtsteilen angegeben. Der NCO-Gehalt wurde nach DIN EN ISO 14896 ermittelt.

Die Bestimmung der Viskosität der Prepolymere erfolgte mit einem Rotationsviskosimeter VT500 der Firma Haake nach DIN EN ISO 3219.

**Tabelle 1**

| **Prepolymer** | 1 | 2 | 3 | 4 | 5 | 6 | 7 | 8 | 9 | 10¹⁾ | 11 |
|---|---|---|---|---|---|---|---|---|---|---|---|
| **NCO-Gehalt** | 8,7% | 12,2% | 6,3% | 4,6% | 12,1% | 8,2% | 6,5% | 4,6% | 8,3% | 7,2% | 8,3 %** |
| **Iso 1 [%]** | 31,3 | 42 | 2620,6 | | | | | 31,3 | 29 | 31,3 | |
| **Iso 2** | | | | | 42,1 | 31,3 | 26 | 20,6 | | | |
| **Hilfsmittel 1** | 0,01 | 0,01 | 0,01 | 0,01 | 0,01 | 0,01 | 0,01 | 0,01 | 0,01 | 0,01 | 0,01 |
| **Polyol 1** | 68,69 | 57,99 | 73,99 | 79,39 | 57,89 | 68,69 | 73,99 | 79,39 | | | |
| **Polyol 2** | | | | | | | | | 68,69 | | |
| **Polyol 3** | | | | | | | | | | 70,99 | |
| **Polyol 4** | | | | | | | | | | | 68,69* |

| | | | | | | | | | | | |
|---|---|---|---|---|---|---|---|---|---|---|---|
| 10 und 11 =Vergleichsbeispiele, Bsp 11: Polyol 4 ist fest, Schmp: 35 °C | | | | | | | | | | | |

Aus den Prepolymeren wurden gemäß den Tabellen 2 bis 5 durch Vermischen mit einer wässrigen Komponente Schäume hergestellt, dabei wurde wie folgt vorgegangen:
In einem kleinen PE-Becher (V=160mL) wird die Wasserkomponente bei Raumtemperatur vorgelegt. Das Isocyanat wird bei Raumtemperatur in dem vorbereiteten Becher (V= 550mL) vorgelegt. Der Vollrath Rührer ist auf die kleinste Drehzahl eingestellt. Für das Vermischen wird ein Einwegrührer mit einem Durchmesser von 65mm genutzt. Die Wasserkomponente wird nun zügig in den transparenten Becher mit dem Isocyanat überführt. Mit dem Rührbeginn wird auch die Stoppuhr gestartet. Die Einsatzstoffe werden mit steigender Drehzahl vermischt, bis ein homogenes Gemisch entsteht. Die Rührzeit ist abhängig von der Reaktivität des Systems und soll maximal 80% der Startzeit betragen.

**Tabelle 2:**

| | Beispiel 1 | Beispiel 2 | Beispiel 3 | Beispiel 4 | Beispiel 5 | Beispiel 6 | Beispiel 7 |
|---|---|---|---|---|---|---|---|
| wässrige Komponente (1) | 15 | 18,75 | 25 | 30 | 37,5 | 45 | 50 |
| Prepolymer 1 (2) | 60 | 56,25 | 50 | 45 | 37,5 | 30 | 25 |
| Mischungsverhältnis (1):(2) | 1:4 | 1:3 | 1:2 | 1:1,5 | 1:1 | 1 :0,67 | 1:0,5 |
| Dichte [g/L] | 91 | 74 | 94 | 106 | 140 | 188 | 220 |
| Wasseraufnahme [g Wasser pro 1g Schaum] | 3,1 | 10,4 | 15,0 | 14,1 | 10,9 | 8,0 | 7,1 |

Tabelle 2 zeigt, dass ein Mischungsverhältnis von wässriger Komponente (1) zu Prepolymer (2) von 1 : 1 zu den besten Wasseraufnahmefähigkeiten führt,

Gemäß Tabellen 3 und 4 wurden die wässrige Komponente und das Prepolymer in Gewichtsverhältnis 1 : 2 vermischt, um einen Schaumstoff herzustellen. Dabei wurden in Tabelle 3 der NCO-Gehalt des Isocyanats variiert und in Tabelle 4 unterschiedliche Polyole zur Herstellung des Prepolymers eingesetzt. In den Tabellen 3 und 4 sind die Dichte und die Wasseraufnahmefähigkeit der erhaltenen Schaumstoffe angegeben.

**Tabelle 3**

| | Beispie l 8 | Beispie l 9 | Beispiel 10 | Beispiel 11 | Beispiel 12 | Beispiel 13 | Beispiel 14 | Beispiel 15 |
|---|---|---|---|---|---|---|---|---|
| wässrige Komponente | 25 | 25 | 25 | 25 | 25 | 25 | 25 | 25 |
| Prepolymer 2, | 50 | | | | | | | |
| NCO=12,2 | | | | | | | | |
| Prepolymer 1 | | 50 | | | | | | |
| NCO=8,7 | | | | | | | | |
| Prepolymer 3 | | | 50 | | | | | |
| NCO=6,3 | | | | | | | | |
| Prepolymer 4 | | | | 50 | | | | |
| NCO=4,6 | | | | | | | | |
| Prepolymer 5 | | | | | 50 | | | |
| NCO=12,1 | | | | | | | | |
| Prepolymer 6 | | | | | | 50 | | |
| NCO=8,2 | | | | | | | | |
| Prepolymer 7 | | | | | | | 50 | |
| NCO=6,5 | | | | | | | | |
| Prepolymer 8 | | | | | | | | 50 |
| NCO=4,6 | | | | | | | | |
| Dichte [g/L] | 88 | 103 | 114 | 573* | 76 | 90 | 132 | 544 |
| Wasseraufnahme | 11,4 | 14,9 | 12,2 | 0,2 | 5,2 | 17,2 | 11,4 | 0,3 |

| | | | | | | | | |
|---|---|---|---|---|---|---|---|---|
| [g Wasser pro 1g Schaum] * grobe Zellstruktur, sehr hart | | | | | | | | |

Tabelle 3 zeigt, dass optimale Wasseraufnahmen bei einem Isocyanatgehalt des Prepolymers von 6- bis 9 Gew-% erhalten werden.

**Tabelle 4**

| | Beispiel 16 | Beispiel 17 | Beispiel 18 | Beispiel 19 |
|---|---|---|---|---|
| wässrige Komponente | 25 | 25 | 25 | 25 |
| Prepolymer 1 | 50 | | | |
| NCO=8,7% | | | | |
| Prepolymer 9 | | 50 | | |
| NCO=8,9% | | | | |
| Prepolymer 10 | | | 50 | |
| NCO=7,2% | | | | |
| Prepolymer 11 | | | | 50 |
| NCO=8,3% | | | | |
| Dichte [g/L] | 103 | 108 | 135 | 109 |
| Wasseraufnahme [g Wasser pro 1g Schaum] | 14,9 | 14,0 | 4,7 | 8,3 |
| | | | .,. | |

## Patentansprüche

1. Verfahren zur Herstellung eines hydrophilen Polyurethanweichschaumstoffs, bei dem man
(a) mindestens ein Polyurethanprepolymer mit einem Isocyanatgehalt von 6 bis 10 Gew.-%, bezogen auf das Gewicht des Isocyanatprepolymers (a), mit
(b) mindestens einer wässrigen Komponente
im Gewichtsverhältnis Polyurethanprepolymer (a) zu wässriger Komponente (b) von 3 zu 1 bis 1 zu 1,2 vermischt und zum Polyurethanweichschaumstoff ausreagieren lässt,
wobei das Polyurethanprepolymer (a) erhältlich ist durch Vermischen und Umsetzen mindestens eines Isocyanats (a1) mit mindestens einem Polyetherol (a2),
das Isocyanat (a1) Methylendiphenylendiisocyanat enthält und
das Polyetherol (a2) eine Hydroxylzahl von 30 bis 60 mg KOH/g aufweist, erhältlich ist durch Alkoxylierung mindestens eines difunktionellen und/oder trifunktionellen Startermoleküls mit Ethylenoxid und Propylenoxid und der Gehalt an Ethylenoxid, bezogen auf das Gesamtgewicht an Alkylenoxid, mindestens 60 Gew.-%, bezogen auf das Gesamtgewicht des Polyetherpolyols (a2), beträgt, wobei das Polyetherol (a2) erhältlich ist durch Propoxylierung des oder der Startermoleküle in einem ersten Schritt bis zu einer Hydroxylzahl von 400 bis 1200 mg KOH/g, anschließend einer Alkoxylierung des propoxylierten Startermoleküls mit einer Mischung von Ethylenoxid und Propylenoxid und schließlich einer Ethoxylierung des so erhaltene Alkoxylierungsprodukts mit 2 bis 10 Gew.-% Ethylenoxid, bezogen auf das zur Herstellung des Polyetherols (a2) eingesetzte Alkylenoxid, sowie
die wässrige Komponente (b) mindestens 90 Gew.-% Wasser (b1) und bis zu 10 Gew.-% nicht silikonhaltige oberflächenaktive Substanzen (b2) enthält, jeweils bezogen auf das Gesamtgewicht der Komponente (b).

2. Verfahren nach Anspruch 1, **dadurch gekennzeichnet, dass** der Isocyanatgehalt des Isocyanatprepolymers (a) 6 bis 9 Gew.-% bezogen auf das Gewicht des Isocyanatprepolymers (a), beträgt.

3. Verfahren nach Anspruch 1 oder 2, **dadurch gekennzeichnet, dass** das Isocyanat (a) 30 bis 100 Gew.-% 4,4' Methylendiphenylendiisocyanat und 0 bis 70 Gew.-% 2,4'-Methylendiphenylendiisocyanat, jeweils bezogen auf das Gesamtgewicht des Isocyanats (a), enthält.

4. Verfahren nach einem der Ansprüche 1 bis 3, **dadurch gekennzeichnet, dass** das Isocyanat (a) weniger als 10 Gew.-% Isocyanate enthält, die von 4,4' Methylendiphenylendiisocyanat und 2,4'-Methylendiphenylendiisocyanat verschieden sind.

5. Verfahren nach einem der Ansprüche 1 bis 4, **dadurch gekennzeichnet, dass** das Isocyanat (a) weniger als 1 Gew.-% 2,2' Methylendiphenylendiisocyanat enthält.

6. Verfahren nach einem der Ansprüche 1 bis 5, **dadurch gekennzeichnet, dass** Gewichtsverhältnis Polyurethanprepolymer (a) zu wässriger Komponente (b) von 2,5 zu 1 bis 1,2 zu 1 beträgt.

7. Verfahren nach einem der Ansprüche 1 bis 6, **dadurch gekennzeichnet, dass** die Umsetzung von Polyurethanprepolymer (a) und wässriger Komponente (b) zum Polyurethanweichschaumstoff in Abwesenheit von tertiären Aminen erfolgt.

8. Verfahren nach einem der Ansprüche 1 bis 7, **dadurch gekennzeichnet, dass** das Polyetherol (a2) einen Gehalt an primären Hydroxylgruppen von 95 bis 100 % aufweist.

9. Verfahren nach einem der Ansprüche 1 bis 8, **dadurch gekennzeichnet, dass** das Startermolekül zur Herstellung des Polyetherols (a2) ausgewählt ist aus der Gruppe, bestehend aus Ethylenglycol, Diethylenglycol, Propylenglykol, Dipropylenglykol, Glycerin, Trimethylolpropan und Mischungen daraus.

10. Verfahren nach einem der Ansprüche 1 bis 9, **dadurch gekennzeichnet, dass** das Polyetherol (a2) erhältlich ist durch Propoxylierung eines Startermolekül in einem ersten Schritt bis zu einer Hydroxylzahl 450 bis 800 mg KOH/g, anschließend mit einer Alkoxylierung mit einer Mischung von Ethylenoxid und Propylenoxid und einer Ethoxylierung des so erhaltenen Alkoxylierungsprodukts mit 3 bis 5 Gew.-% Ethylenoxid, bezogen auf das zur Herstellung des Polyetherols (a2) eingesetzte Alkylenoxid.

11. Verfahren nach einem der Ansprüche 1 bis 10, **dadurch gekennzeichnet, dass** zur Herstellung des Polyisocyanatprepolymers (a) neben dem Polyetherol (a2) keine weiteren Verbindungen mit gegenüber Isocyanat reaktiven Gruppen eingesetzt werden.

12. Verfahren nach einem der Ansprüche 1 bis 11, **dadurch gekennzeichnet, dass** die Dichte des Polyurethanweichschaumstoffs 80 bis 120 g/L beträgt.

13. Hydrophiler Polyurethanweichschaumstoff, erhältlich nach einem Verfahren gemäß einem der Ansprüche 1 bis 12.

14. Verwendung eines hydrophilen Polyurethanweichschaumstoffs gemäß Anspruch 13 zur Wundbehandlung.

## Claims

1. A process for producing a hydrophilic flexible polyurethane foam, in which
(a) at least one polyurethane prepolymer having an isocyanate content of 6 to 10% by weight, based on the weight of the isocyanate prepolymer (a), is mixed with
(b) at least one aqueous component in a ratio by weight of polyurethane prepolymer (a) to aqueous component (b) from 3:1 to 1:1.2 and allowed to react to form the flexible polyurethane foam,
wherein the polyurethane prepolymer (a) is obtainable by mixing and reacting at least one isocyanate (a1) with at least one polyetherol (a2), the isocyanate (a1) comprises methylenediphenylene diisocyanate and
the polyetherol (a2) has a hydroxyl number of 30 to 60 mg KOH/g and is obtainable by alkoxylating at least one difunctional and/or trifunctional starter molecule with ethylene oxide and propylene oxide and the ethylene oxide content, based on the total weight of alkylene oxide, is at least 60% by weight, based on the total weight of the polyether polyol (a2), wherein the polyetherol (a2) is obtainable by propoxylation of the starter molecule(s) in a first step up to a hydroxyl number of 400 to 1200 mg KOH/g, followed by an alkoxylation of the propoxylated starter molecule with a mixture of ethylene oxide and propylene oxide and finally an ethoxylation of the alkoxylation product thus obtained with 2 to 10% by weight ethylene oxide, based on the alkylene oxide used for producing the polyetherol (a2), and
the aqueous component (b) comprises at least 90% by weight water (b1) and up to 10% by weight non-silicone-containing surface-active substances (b2), based in each case on the total weight of component (b) .

2. The process according to claim 1, wherein the isocyanate content of the isocyanate prepolymer (a) is 6 to 9% by weight, based on the weight of the isocyanate prepolymer (a).

3. The process according to claim 1 or 2, wherein the isocyanate (a) comprises 30 to 100% by weight 4,4'-methylenediphenylene diisocyanate and 0 to 70% by weight 2,4'-methylenediphenylene diisocyanate, based in each case on the total weight of the isocyanate (a).

4. The process according to any of claims 1 to 3, wherein the isocyanate (a) comprises less than 10% by weight isocyanates which are different from 4,4'-methylenediphenylene diisocyanate and 2,4'-methylenediphenylene diisocyanate.

5. The process according to any of claims 1 to 4, wherein the isocyanate (a) comprises less than 1% by weight 2,2'-methylenediphenylene diisocyanate.

6. The process according to any of claims 1 to 5, wherein the ratio by weight of polyurethane prepolymer (a) to aqueous component (b) is from 2.5:1 to 1.2:1.

7. The process according to any of claims 1 to 6, wherein polyurethane prepolymer (a) and aqueous component (b) are reacted to form the flexible polyurethane foam in the absence of tertiary amines.

8. The process according to any of claims 1 to 7, wherein the polyetherol (a2) has a primary hydroxyl group content of 95 to 100%.

9. The process according to any of claims 1 to 8, wherein the starter molecule for producing the polyetherol (a2) is selected from the group consisting of ethylene glycol, diethylene glycol, propylene glycol, dipropylene glycol, glycerol, trimethylolpropane and mixtures thereof.

10. The process according to any of claims 1 to 9, wherein the polyetherol (a2) is obtainable by propoxylation of a starter molecule in a first step up to a hydroxyl number of 450 to 800 mg KOH/g, followed by an alkoxylation with a mixture of ethylene oxide and propylene oxide and an ethoxylation of the alkoxylation product thus obtained with 3 to 5% by weight ethylene oxide, based on the alkylene oxide used to produce the polyetherol (a2).

11. The process according to any of claims 1 to 10, wherein, in addition to the polyetherol (a2), no further compounds with isocyanate-reactive groups are used to produce the polyisocyanate prepolymer (a).

12. The process according to any of claims 1 to 11, wherein the density of the flexible polyurethane foam is 80 to 120 g/L.

13. A hydrophilic flexible polyurethane foam obtainable by a process according to any of claims 1 to 12.

14. The use of a hydrophilic flexible polyurethane foam according to claim 13 for treating wounds.

## Revendications

1. Procédé pour la production d'une mousse souple de polyuréthane hydrophile, dans lequel on mélange
(a) au moins un prépolymère polyuréthane ayant une teneur en isocyanate de 6 à 10 % en poids, par rapport au poids du prépolymère d'isocyanate (a), avec
(b) au moins un composant aqueux
en le rapport en poids prépolymère polyuréthane (a) à composant aqueux (b) de 3 : 1 à 1 : 1,2 et on fait réagir complètement ce mélange pour aboutir à la mousse souple de polyuréthane,
dans lequel le prépolymère polyuréthane (a) peut être obtenu par mélange et mise en réaction d'au moins un isocyanate (a1) avec au moins un polyétherol (a2), l'isocyanate (a1) contient du diisocyanate de méthylènediphénylène et
le polyétherol (a2) présente un indice d'hydroxyle de 30 à 60 mg de KOH/g, peut être obtenu par alcoxylation d'au moins une molécule de départ difonctionnelle et/ou une molécule de départ trifonctionnelle avec de l'oxyde d'éthylène et de l'oxyde de propylène et la teneur en oxyde d'éthylène, par rapport au poids total d'oxyde d'alkylène, vaut au moins 60 % en poids par rapport au poids total du polyétherpolyol (a2), le polyétherol (a2) pouvant être obtenu par propoxylation de la ou des molécules de départ dans une première étape jusqu'à un indice d'hydroxyle de 400 à 1 200 mg de KOH/g, ensuite une alcoxylation de la molécule de départ propoxylée à l'aide d'un mélange d'oxyde d'éthylène et d'oxyde de propylène, et enfin une éthoxylation du produit d'alcoxylation ainsi obtenu à l'aide de 2 à 10 % en poids d'oxyde d'éthylène, par rapport à l'oxyde d'alkylène utilisé dans la préparation du polyétherol (a2), et
le composant aqueux (b) contient au moins 90 % en poids d'eau (b1) et jusqu'à 10 % en poids de substances tensioactives (b2) ne contenant pas de silicone, chaque fois par rapport au poids total du composant (b).

2. Procédé selon la revendication 1, **caractérisé en ce que** la teneur en isocyanate du prépolymère d'isocyanate (a) vaut 6 à 9 % en poids par rapport au poids du prépolymère d'isocyanate (a).

3. Procédé selon la revendication 1 ou 2, **caractérisé en ce que** l'isocyanate (a) contient 30 à 100 % en poids de 4,4'-diisocyanate de méthylènediphénylène et 0 à 70 % en poids de 2,4'-diisocyanate de méthylènediphénylène, chaque fois par rapport au poids total de l'isocyanate (a).

4. Procédé selon l'une quelconque des revendications 1 à 3, **caractérisé en ce que** l' isocyanate (a) contient moins de 10 % en poids d'isocyanates qui sont différents du 4,4'-diisocyanate de méthylènediphénylène et du 2,4'-diisocyanate de méthylènediphénylène.

5. Procédé selon l'une quelconque des revendications 1 à 4, **caractérisé en ce que** l' isocyanate (a) contient moins de 1 % en poids de 2,2'-diisocyanate de méthylènediphénylène.

6. Procédé selon l'une quelconque des revendications 1 à 5, **caractérisé en ce que** le rapport en poids de prépolymère polyuréthane (a) à composant aqueux (b) vaut de 2,5 : 1 à 1,2 : 1.

7. Procédé selon l'une quelconque des revendications 1 à 6, **caractérisé en ce que** la réaction de prépolymère polyuréthane (a) et composant aqueux (b) conduisant à la mousse souple de polyuréthane s'effectue en absence d'amines tertiaires.

8. Procédé selon l'une quelconque des revendications 1 à 7, **caractérisé en ce que** le polyétherol (a2) présente une teneur en groupes hydroxy primaires de 95 à 100 % en poids.

9. Procédé selon l'une quelconque des revendications 1 à 8, **caractérisé en ce que** la molécule de départ dans la préparation du polyétherol (a2) est choisie dans le groupe constitué par l'éthylèneglycol, le diéthylèneglycol, le propylèneglycol, le dipropylène-glycol, le glycérol, le triméthylolpropane et des mélanges de ceux-ci.

10. Procédé selon l'une quelconque des revendications 1 à 9, **caractérisé en ce que** le polyétherol (a2) peut être obtenu par propoxylation d'une molécule de départ dans une première étape jusqu'à un indice d'hydroxyle de 450 à 800 mg de KOH/g, ensuite par une alcoxylation à l'aide d'un mélange d'oxyde d'éthylène et d'oxyde de propylène, et une éthoxylation du produit d'alcoxylation ainsi obtenu à l'aide de 3 à 5 % en poids d'oxyde d'éthylène, par rapport à l'oxyde d'alkylène utilisé dans la préparation du polyétherol (a2) .

11. Procédé selon l'une quelconque des revendications 1 à 10, **caractérisé en ce que** dans la préparation du prépolymère de polyisocyanate (a) outre le polyétherol (a2) aucun autre composé à groupes réactifs vis-à-vis d'isocyanate n'est utilisé.

12. Procédé selon l'une quelconque des revendications 1 à 11, **caractérisé en ce que** la densité de la mousse souple de polyuréthane vaut 80 à 120 g/l.

13. Mousse souple de polyuréthane hydrophile, pouvant être obtenue conformément à un procédé selon l'une quelconque des revendications 1 à 12.

14. Utilisation d'une mousse souple de polyuréthane hydrophile selon la revendication 13 pour le traitement de plaies.
